# EUROPEAN PATENT APPLICATION

(11) **EP 4 299 576 A1**
(43) Date of publication of application: **03.01.2024**
(21) Application number: 22182177.0
(22) Date of filing: 30.06.2022
(51) Int. Cl.: C07D 487/04, H01L 51/50

(54) **[1,2,4]-TRIAZOLO[A]PYRIMIDINE DERIVATIVES AS THERMALLY ACTIVATED DELAYED FLUORESCENCE (TADF) EMITTERS AND PROCESS OF PREPARATION THEREOF**

(71) Applicant: Vilnius University, 01513 Vilnius (LT); Tikslioji sinteze, UAB, 08310 Vilnius (LT)
(72) Inventor: Tumkevicius, Sigitas, LT-06102 Vilnius (LT); Serevicius, Tomas, LT-03212 Vilnius (LT); Fiodorova, Irina, LT-03202 Vilnius (LT); Adomenas, Povilas, LT-08311 Vilnius (LT); Skaisgiris, Rokas, LT-11311 Vilnius (LT); Jursenas, Saulius, Vilnius (LT)
(74) Representative: Petniunaite, Jurga

(57) **Abstract**

The present disclosure pertains to a novel organic thermally activated delayed fluorescence (TADF) compound selected from a group of compounds consisting of [1,2,4]-triazolo[1,5-a]pyrimidine and [1,2,4]-triazolo[4,3-a]pyrimidine derivatives. The structure of compounds disclosed comprises [1,2,4]-triazolo[1,5-a]pyrimidine and [1,2,4]-triazolo[4,3-a]pyrimidine heterocycles which are modified at the position 6 with alkyl groups that enhance steric hindrance of an acceptor unit with donor unit or phenylene spacer. This structural modification: reduces the conjugation between structural parts of emitter, shifts the absorption and fluorescence bands to a blue region of spectra, and enables better separation of frontier molecular orbitals. Moreover, a novel class of TADF emitters comprising [1,2,4]-triazolo[4,3-a]pyrimidine acceptor unity has been developed. The process of preparation of TADF compounds of the invention comprises at least a step of modification of [1,2,4]-triazolo[1,5-a]pyrimidine and [1,2,4]-triazolo[4,3-a]pyrimidine heterocycles at the position 6 in the initial steps of the process, and a step of formation of [1,2,4]-triazolo[4,3-a]pyrimidine moiety from corresponding 2-methylsulfonylpyrimidines by three-step one-pot process of preparation. According to the present invention, the organic molecules exhibit emission maxima in the blue, sky-blue or green range. The photoluminescence quantum yields of the organic molecules according to the present invention are, in particular, more than 24%. The molecules according to the invention exhibit in particular thermally activated delayed fluorescence.

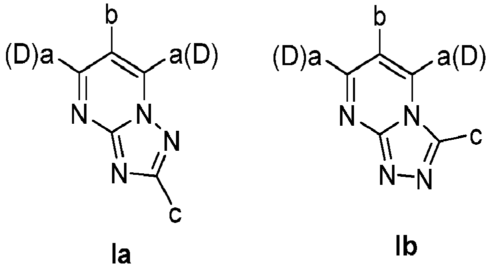

## Description

### FIELD OF INVENTION

The present invention relates to the provision of Thermally Activated Delayed Fluorescence (TADF) compounds for use in organic light emitting diodes or other electroluminescent devices.

### BACKGROUND ART

Organic light emitting diodes are highly attractive for display and lighting applications due to the high efficiency, low production cost and flexibility. However, according to the spin statistics in OLED devices, only 25% of excitons are bright singlets, limiting the device efficiency. Utilization of non-emissive triplet excitons can lead to nearly 100% internal quantum efficiency (IQE). Thus, the maximum IQE of commercial devices is 25% for OLEDs using conventional fluorescent emitters, and increases to 100% for heavy metal employing phosphorescent emitters. The phosphorescent emitters usually consist of organometallic compounds containing rare noble metals. However, employing noble metals such as iridium (Ir) or platinum (Pt) in phosphorescent emitters is problematic in view of their scarcity and cost.

Therefore, development of new and alternative efficient emitters is highly warranted and important for production of efficient and low cost OLEDs.

Since 2012 novel type of organic emitters based on thermally activated delayed fluorescence (TADF) has been developed. TADF emitters are able to convert excitons in the lowest triplet excited state (T1) to the lowest singlet excited state (S1) via reverse intersystem crossing (RISC) using thermal energy. Thus, TADF materials can harvest light by using both triplet and singlet excitons, so IQE 100% could be achieved without use of noble metals.

Among the approaches that are suggested to enable TADF, the most popular is based on constructing TADF compounds from electron-donating (D) and electron-accepting (A) fragments. Typically, the TADF compounds are of a donor-acceptor (D-A) or a donor-acceptor-donor architecture. Building the molecular compounds of D and A units decouples HOMO and LUMO and enables triplet upconversion. The efficient separation of frontier molecular orbitals could be achieved either by twisting D and A units toward orthogonality or by keeping the molecular structure rather flat, but with extended conjugation in the HOMO and LUMO. The decoupling of HOMO and LUMO also depends on the electron withdrawing and electron donating strength of A and D molecular units.

There are known various structures comprised in TADF compounds. Among them, aromatic and heterocyclic amines, such as diphenylamine, carbazole, phenoxazine, phenothiazine, 9,9-dimethyl-9,10-dihydroacridine and their derivatives constitute preferred donor groups used to construct TADF materials. Among acceptor unities in TADF compounds, various π-defficient heterocycles, such as pyridine, oxadiazole, triazole, thiazole, imidazole, pyridazine, pyrimidine, pyrazine or electron withdrawing groups, such as cyano, ester, or sulfonyl, are often selected to build up the TADF materials.

Patent literature also describes various TADF emitters. An international patent application WO2018080067 describes organic TADF compound structure of which composes from phenoxazine, phenothiazine or carbazole donor moieties and nitrogen heterocycle acceptor unit, such as pyrimidine or triazine.

Patent US11201295B2 describes organic compounds being TADF emitters. The compounds consist of various widely used donor moieties and nitrogen containing heterocyclic acceptor moieties functionalized with electron withdrawing arylsulfonyl groups, which strengthen the electron accepting properties of nitrogen heterocycle, such as pyridine, chinoline, benzimidazole, benzotiazole, triazole, etc. Nonetheless, the described TADF compounds contain neither [1,2,4]-triazolo[1,5-a]pyrimidine nor [1,2,4]-triazolo[4,3-a]pyrimidine heterocycles as acceptor units.

An international patent application WO2019/046734A1 describes pyridine-based organic TADF compounds based on an acceptors consisting of variably substituted pyridine nucleus. The compounds described therein are capable of luminescing at higher excitation states. Thereby, the described TADF compounds contain neither [1,2,4]-triazolo[1,5-a]pyrimidine nor [1,2,4]-triazolo[4,3-a]pyrimidine heterocyclic moieties as acceptors, though monocyclic heterocycles - pyrimidine and [1,2,4]-triazole are extensively employed to build up efficient TADF emitters.

[1,2,4]-Triazolo[1,5-a]pyrimidine heterocyclic moiety in a combination with phenoxazine donor unit and phenylene spacer was used for fabrication yellow OLEDs (X. Zeng, T. Zhou, J. Liu, K. Wu, S. Li, X. Xiao, Y. Zhang, S. Gong, G. Xie, and C Yang. Advanced Optical Materials, 2018, 6, 1801071; DOI: 10.1002/adom.201801071).

As follows from the above-mentioned literature, donor structures as well as acceptor structures, forming up TADF compounds, undergo continuous development for using in various applications, including OLEDs. Therefore, despite the progress made there is a need to provide alternative compounds (especially blue and sky-blue emitters) for use in display and lighting applications. Although many blue phosphorescent materials have been developed, they suffer from stability as blue color requires higher energy excited states which accelerate the degradation process of OLEDs.

### SUMMARY OF THE INVENTION

**Problem.** There is a need of alternative compounds (blue and sky-blue emitters) for use in display and lighting applications. Although many blue phosphorescent materials have been developed, they suffer from stability as blue color requires higher energy excited states which accelerate the degradation process of OLEDs.

**Solution by the invention.** The structure of compounds described herein differs from the reported in a literature:
- [1,2,4]-triazolo[1,5-a]pyrimidine heterocycle is modified at the position 6 with alkyl groups that enhances steric hindrance of an acceptor unit with donor unit or phenylene spacer.
- [1,2,4]-triazolo[4,3-a]pyrimidine heterocycle for the first time is used as an acceptor unit in TADF compounds.
- [1,2,4]-triazolo[4,3-a]pyrimidine heterocycle is modified at the position 6 with alkyl groups that enhances steric hindrance of an acceptor unit with donor unit or phenylene spacer.

This structural modification:
- reduces the conjugation between structural parts of emitter,
- shifts the absorption and fluorescence bands to a blue region of spectra,
- and enables better separation of frontier molecular orbitals.

Moreover, a novel class of TADF emitters comprising [1,2,4]-triazolo[4,3-a]pyrimidine acceptor unity has been developed.

According to the present invention, the organic molecules exhibit emission maxima in the blue, sky-blue or green range. The photoluminescence quantum yields of the organic molecules according to the present invention are, in particular, more than 24%. The molecules according to the invention exhibit in particular thermally activated delayed fluorescence (TADF).

**TADF compounds by the invention.** The present invention provides an organic TADF compound selected from the groups of compounds according to formula **Ia** and **Ib**: 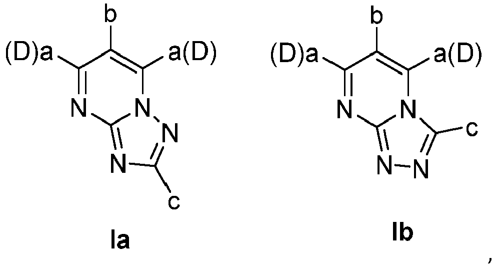 wherein:
- -a(D) are group containing at least one donor group D,
- -c and -b are other groups.

As discussed further below and with reference to some specific embodiments both [1,2,4]-triazolo[1,5-a]pyrimidine (formula la) and [1,2,4]-triazolo[4,3-a]pyrimidine (formula Ib) containing acceptor groups of the invention can be used to provide TADF compounds with useful properties for OLEDs, for example to provide light emitting materials.

More generally, the group bonded at position -b for compounds of formula Ia or formula **Ib** may be selected from the group consisting of:
- -H, substituted or unsubstituted primary, secondary or tertiary alkyl, that may be cyclic and may be unsaturated, for example C1-C10.

The group bonded at position -c for compounds of formula Ia or formula Ib may be selected from the group consisting of:
- -H, substituted or unsubstituted primary, secondary or tertiary alkyl, that may be cyclic and may be unsaturated, for example C1-C10; substituted or unsubstituted aryl.
   - a(D) in formula **Ia** or formula **Ib** can each independently be a substituted or unsubstituted N-heteroaromatic donor group (D) bonded to [1,2,4]-triazolo[1,5-a]pyrimidine (IIa) or [1,2,4]-triazolo[4,3-a]pyrimidine (IIb) nucleus directly via a heteroatom N.

Thus, the TADF compounds of the invention may be selected from the group consisting of compounds according to formula **IIa** and formula **IIb**.

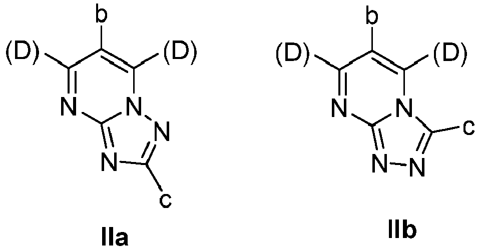

Donor group (D) may be selected from N-heteroaromatics consisting of substituted or unsubstituted carbazole or substituted or unsubstituted 9,10-dihydroacridane.

Groups at positions -b or -c has the same meaning as in formula **Ia** and **Ib**.
- a(D) in formula Ia or formula Ib can each independently be a substituted or unsubstituted N-heteroaromatic donor group (D) bonded to [1,2,4]-triazolo[1,5-a]pyrimidine (IIIa) or [1,2,4]-triazolo[4,3-a]pyrimidine (IIIb) nucleus via a substituted of unsubstituted aromatic ring.

Thus, the TADF compounds of the invention may be selected from the group consisting of compounds according to formula **IIIa** and formula **IIIb**. 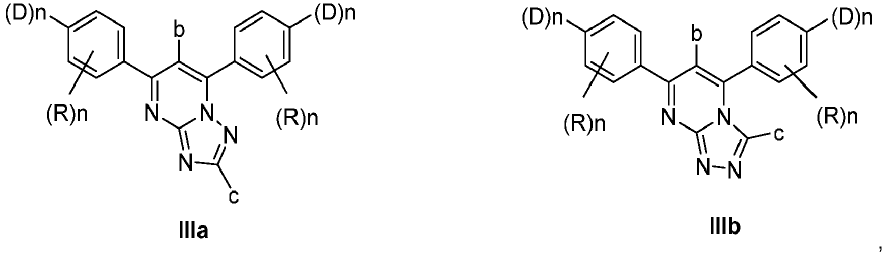 wherein
- (D)n- denotes the presence of n donor groups each bonded to carbon atom of benzene ring; wherein n is at least 1 for one of the rings;
Donor groups D has the same meaning as for formula IIa and formula IIb;

Where donor groups are present on an aryl group of a TADF compound of the invention the number n may be 1, 2, 3 etc. The maximum number of groups D possible on an aryl group being determined by the number of bonding positions available on the aryl group.

Groups at positions -b or -c has the same meaning as for formula Ia and formula lb.

(R)n denotes the presence of alkyl groups which may be selected from the group consisting of substituted or unsubstituted primary, secondary or tertiary alkyl; wherein n is from 0 to 4 for one of the benzene rings;
A wide range of donor groups D for TADF compounds are known. Donor groups D in a compound of the invention may be selected from: 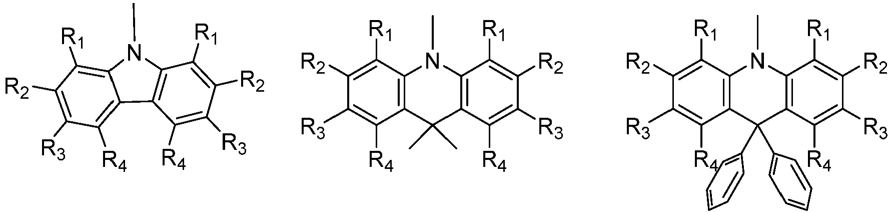 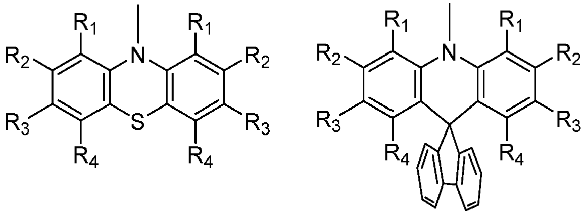 wherein each group R₁, R₂, R₃, and R₄ is, independently for each occurrence, selected from the group consisting of -H, substituted or unsubstituted primary, secondary or tertiary alkyl (for example C1-C10 or even C1-C4); substituted or unsubstituted aryl or heteroaryl, amino and the like.

Where a group R₁, R₂, R₃, or R₄ is amino it may be NH₂, NHR or NR² where the substituents R at the nitrogen may be alkyl (for example, substituted or unsubstituted C1-C10), aryl or heteroaryl. Often each donor group of the compound of the invention is not:

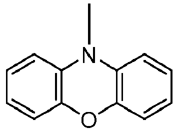

Where carbazole based donor groups D are employed they may be substituted at one or both positions para- to the nitrogen (R₃) while the other positions may be H. The para- (R3) position or positions may be substituted with an alkyl group, for example substituted or unsubstituted primary, secondary or tertiary alkyl (for example, C1-C10 or even C1-C4).

Thus, the donor groups D may be, for example, t-butyl para- (to the nitrogen) substituted, i.e.: 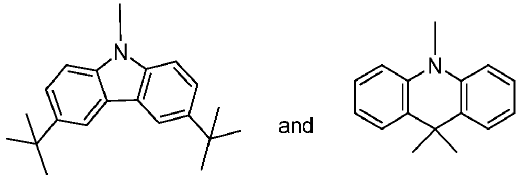 with bonding in TADF compound of the invention via the carbazole and 9,10-dihydrodihydroacridine nitrogen to the acceptor group of formula IIa or formula IIb, or to aryl group of formula IIIa or formula IIIb. [1,2,4]-triazolo[1,5-a]pyrimidine or [1,2,4]-triazolo[4,3-a]pyrimidine containing TADF compounds of the invention (denoted IF294, IF306, IF315, IF319, IF337, IF345) may have a structure from the group consisting of:

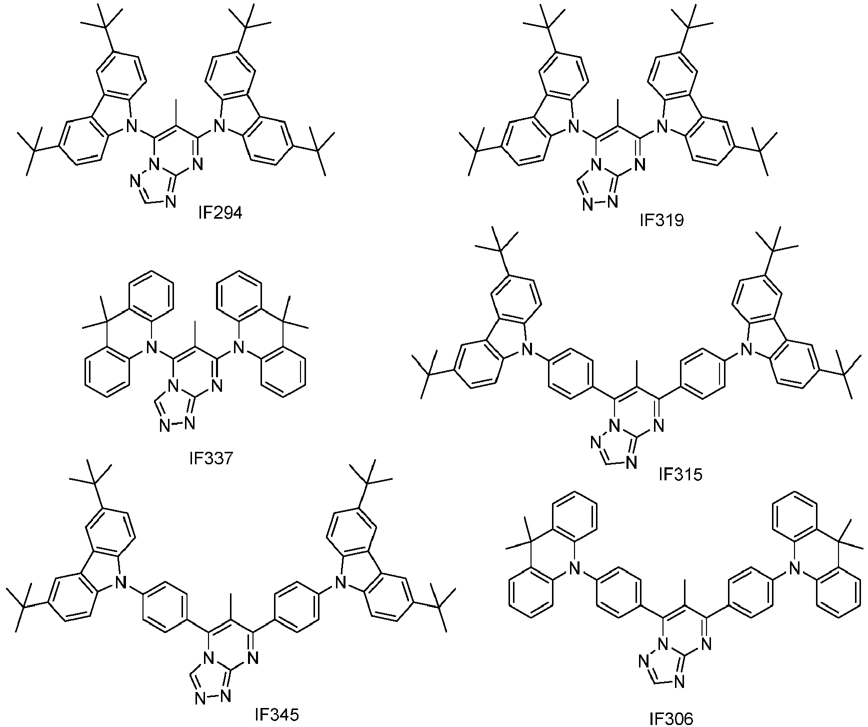

In addition or alternatively, the [1,2,4]-triazolo[1,5-a]pyrimidine or [1,2,4]-triazolo[4,3-a]pyrimidine containing TADF compounds of the invention may not include compounds which contain a donor group of formula:

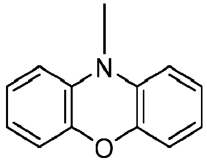

In these examples, where the donor groups are directly attached to [1,2,4]-triazolo[1,5-a]pyrimidine or [1,2,4]triazolo[4,3-a]pyrimidine, or to para position of phenylene spacer, notably good results may be obtained as discussed further below.

Further, useful adjustment of the TADF properties of the compounds may be found, where alkyl groups, such as methyl, on the phenylene spacer as in formula IIIa and formula IIIb can enhance the steric hindrance between the donor D moieties and phenylene spacer. For example, the methyl groups in phenylene spacer are in the ortho position to the donor D groups.

More generally, the TADF compounds of the invention present relatively rigid structures for TADF materials that can be used to manufacture efficient TADF-based OLEDs.

**Synthesis of the TADF compounds.** Examples of synthetic routes are described hereafter and with reference to specific embodiments. A certain specifics of the method or routes for synthesis of TADF compounds is considered in that, the process of preparation comprises at least a step of modification of [1,2,4]-triazolo[1,5-a]pyrimidine and [1,2,4]-triazolo[4,3-a]pyrimidine heterocycles at the position 6 with alkyl groups in the initial steps of the process and formation of [1,2,4]-triazolo[4,3-a]pyrimidine moiety from corresponding 2-methylsulfonylpyrimidines by three-step one-pot process of preparation in the last step of reaction sequences.

The details of manufacturing of TADF compounds of the invention are examplified by the synthesis of IF294, IF306, IF315, IF319, IF337, and IF345 and are disclosed in the further chapter "Detailed Description of Embodiments and Experimental Results".

### BRIEF DESCRIPTION OF DRAWINGS

The drawings are provided as a reference to possible embodiments and are not intended to limit the scope of the invention. Neither of the drawings nor the graphs presented herein should be construed as limiting the scope of the invention, but merely as an example of a possible embodiment.
- **Fig. 1**: depicts the formula of the TADF compound **IF294** and electron density distribution in HOMO and LUMO of the TADF compound **IF294;**
- **Fig. 2**: depicts absorption, fluoprescence and phosphorescence (at 10K) spectra of the TADF compound **IF294;**
- **Fig. 3**: depicts the fluorescence decay transient of TADF compound **IF294;**
- **Fig. 4**: shows absorption, fluoprescence and phosphorescence (at 10 K) spectra of TADF compound **IF306;**
- **Fig. 5**: shows the fluorescence decay transient of TADF compound **IF306;**
- **Fig. 6**: shows absorption, fluoprescence and phosphorescence (at 10 K) spectra of the TADF compound **IF315;**
- **Fig. 7**: Fluorescence decay transient of TADF compound **IF3115;**
- **Fig. 8**: shows absorption, fluoprescence and phosphorescence (at 10 K) spectra of the TADF compound **IF319;**
- **Fig. 9**: Fluorescence decay transient of TADF compound **IF319;**
- **Fig. 10**: shows absorption, fluoprescence and phosphorescence (at 10 K) spectra of the TADF compound **IF337;**
- **Fig. 11**: Fluorescence decay transient of TADF compound **IF337;**
- **Fig. 12**: shows absorption, fluoprescence and phosphorescence (at 10 K) spectra of the TADF compound **IF345;**
- **Fig. 13**: Fluorescence decay transient of TADF compound **IF345;**
- **Fig. 14**: Electroluminescence spectrum of a OLED device with **IF294** as an emitter and its EQE vs Luminance graph;
- **Fig 15**: depicts the principal scheme of reactions employed for the manufacture of [1,2,4]-triazolo[1,5-a]pyrimidines.
- **Fig 16**: depicts the principal scheme of reactions employed for the manufacture of [1,2,4]-triazolo[4,3-a]pyrimidines.

### DETAILED DESCRIPTION OF EMBODIMENTS AND EXPERIMENTAL RESULTS

**General Synthesis information.** Solvents were purified by known procedures. Melting points were determined in open capillaries with a digital melting point IA9100 series apparatus (ThermoFischer Scientific) and were not corrected. Thin layer chromatography was performed using TLC-aluminum sheets with silica gel (Merck 60 F254). Visualization was accomplished by UV light. Column chromatography was performed using Silica gel 60 (0.040-0.063 mm) (Merck). NMR spectra were recorded on a Bruker Ascend 400 (400 MHz and 100 MHz for ¹H and ¹³C, respectively). ¹H NMR and ¹³C NMR spectra were referenced to residual solvent peaks. High Resolution Mass Spectrometry (HRMS) analyses were carried out on a Dual-ESI Q-TOF 6520 (Agilent Technologies) mass spectrometer.

The initial materials used for the manufacture of TADF compounds were synthesized following procedures reported in a literature:
4.6-Dichloro-5-methyl-2-methylthiopyrimidine as in WO2012/110986 A1 (https://worldwide.espacenet.com/patent/search?q=pn%3DWO2012110986A1);
4.6-Diiodo-5-methyl-2-methylthiopyrimidine as in T. Serevičius, R. Skaisgiris, I. Fiodorova, G. Kreiza, D. Banevičius, K. Kazlauskas, S. Tumkevičius S. Juršènas. Journal of Materials Chemistry C, 2021, 9, 836-861 (https://doi.org/10.1039/D0TC05503D)
5,7-Dichloro-6-methyl-[1,2,4]-triazolo[1,5-a]pyrimidine as in T. Saito, T. Obitsu, C. Minamoto, T. Sugiura, N. Matsumura, S. Ueno, A. Kishi, S. Katsumata, H. Nakai, M. Toda. Bioorganic and Medicinal Chemistry, 2011, 19, 5955-5966 (http://dx.doi.ora/10.1016/i.bmc.2011.08.055)

**General Information of Photophysical Measurements.** Absorption spectra of TADF compounds was measured in 10⁻⁵M toluene solution while the rest of photophysical properties were analyzed in PMMA film at 1wt% concentration. Solid-state films were prepared by dissolving each material and host at appropriate ratios in toluene solutions and then wet-casting the solutions on quartz substrates in air surrounding. Absorption spectra were measured using Lambda 950 UV/Vis spectrophotometer (PerkinElmer). Time-integrated, low-temperature phosphorescence spectra (PH) and fluorescence decay transients were measured using nanosecond YAG:Nd³⁺ laser NT 242 (Ekspla, τ = 7 ns, λ=340 nm, pulse energy 200 µJ, repetition rate 1 kHz) and time-gated iCCD camera New iStar DH340T (Andor). Fluorescence transients were obtained by exponentially increasing delay and integration time (C. Rothe, A. P. Monkman, Phys. Rev. B 2003, 68, 075208). Fluorescence quantum yields (Φ_{PL}, ±5% error) of films in air surrounding were estimated using the integrated sphere method (J. C. de Mello, H. F. Wittmann, R. H. Friend, Adv. Mater. 1997, 9, 230) by integrating sphere (Sphere Optics) connected to the CCD spectrometer PMA-12 (Hamamatsu) via optical fiber. Solid-state samples were mounted in closed cycle He cryostat (Cryo Industries 204N) for all measurements in oxygen-free conditions. OLED device were fabricated on precleaned indiumtin oxide (ITO)-coated glass substrates. The small-molecule and cathode layers were thermally evaporated using vacuum evaporation apparatus (Vacuum Systems and Technologies Ltd) at <6 × 10⁻⁶ Torr pressure and deposition rate of about 1Å/s. OLED devices were encapsulated with a clear glass cover to prevent the interaction with ambient. Device current-voltage (*I*-*V*) characteristics and electroluminescence properties were measured using calibrated integrating sphere (Orb Optronics) and CCD spectrometer PMA-11 (Hamamatsu), powered by 2601A power supply unit (Keithley).

**General Information of DFT calculations.** Quantum chemical calculations were performed by using Density Functional Theory (DFT) as implemented in the Gaussian 09 software package at the B3LYP/6-31 G(d) level (M. J. Frisch, G. W. Trucks, H. B. Schlegel, G. E. Scuseria, M. A. Robb M. A, J. R. Cheeseman et. al., Gaussian 09, Gaussian Inc;, Wallingford, CT, 2004). Polarizable Continuum Model (PCM) was used to estimate the solvation behavior of toluene surrounding.

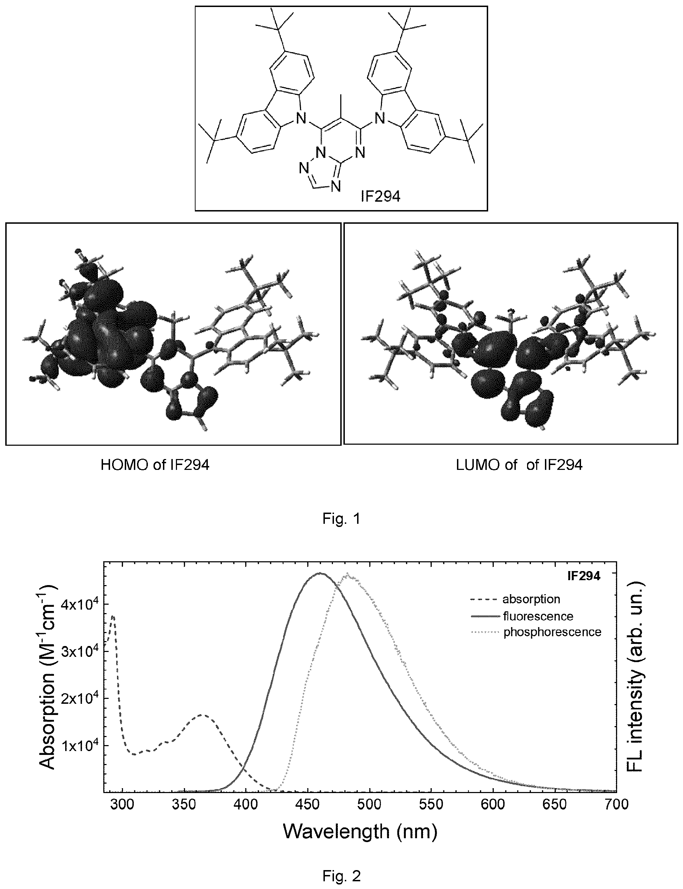

**DFT Calculations Results of Some Embodiments.** DFT calculated ground-state geometry and HOMO-LUMO distribution of the representative compound **IF294** are shown in Fig 1. Compound **IF294,** same as the rest of compounds, possess nearly orthogonal donor (D) - acceptor (A) molecular orientation. Such molecular structure enables the localization of π-electron density over the donor unit in HOMO and over the acceptor unit in LUMO. Carbazole-substituted compounds **IF294/IF319** showed the deepest HOMO levels (down to -5.57 eV), followed by **IF315/IF345** with additional phenyl spacer unit (HOMO energies of -5.33/-5.32 eV) and compounds **IF337/306** with acridine units (HOMO energies as low as -5.23/-5.01 eV). LUMO energies followed the same trend, when the shallowest LUMO was found for **IF294** and the deepest one - to **IF306.** Singlet-triplet energy gaps were in the range of 4-481 meV, when the lowest gap was estimated for compound **IF306.** Details of DFT calculations are enclosed in Table 1.

**Table 1. characteristics of DFT-calculated emission properties of TADF compounds.**

| **TADF compound** | **HOMO (eV)** | **LUMO (eV)** | ***E*_{S1} (eV)** | ***E*_{T1} (eV)** | **Δ*E*_{ST} (meV)** |
|---|---|---|---|---|---|
| **IF294** | -5.56 | -1.91 | 3.041 | 2.733 | 310 |
| **IF306** | -5.01 | -2.34 | 2.242 | 2.238 | 4 |
| **IF315** | -5.33 | -2.02 | 2.906 | 2.677 | 229 |
| **IF319** | -5.57 | -2.02 | 2.961 | 2.480 | 481 |
| **IF337** | -5.23 | -2.21 | 2.232 | 2.203 | 29 |
| **IF345** | -5.32 | -2.07 | 2.833 | 2.430 | 403 |

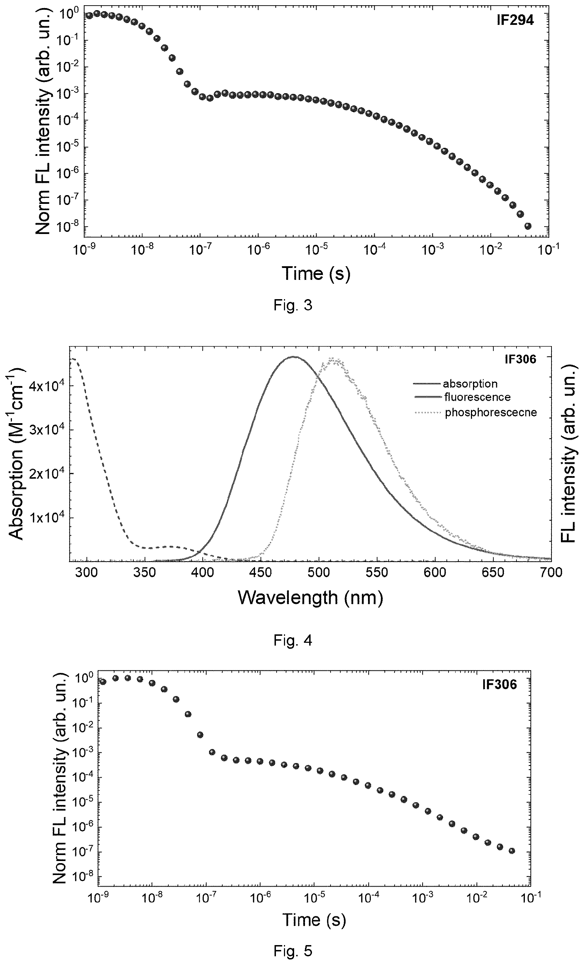

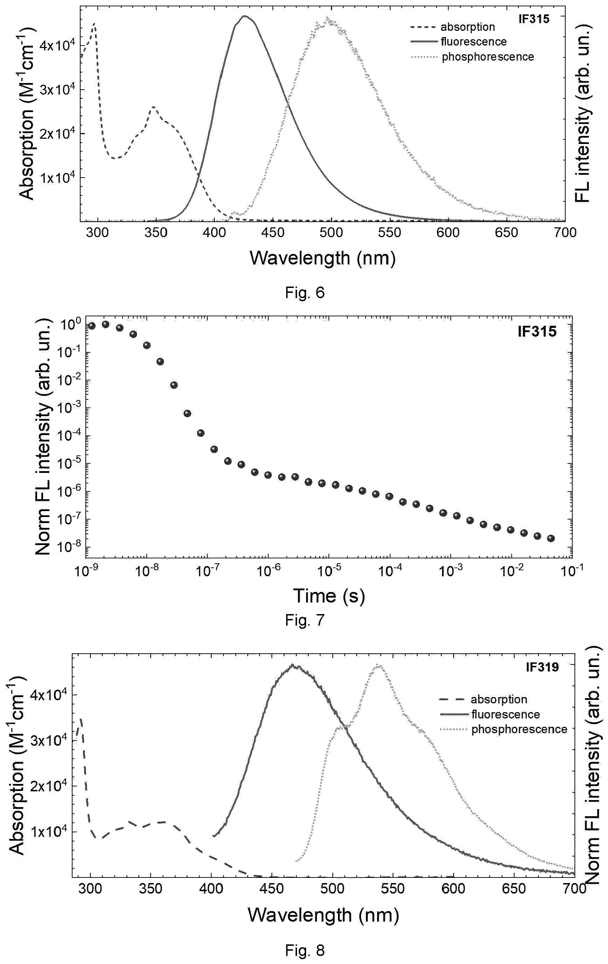

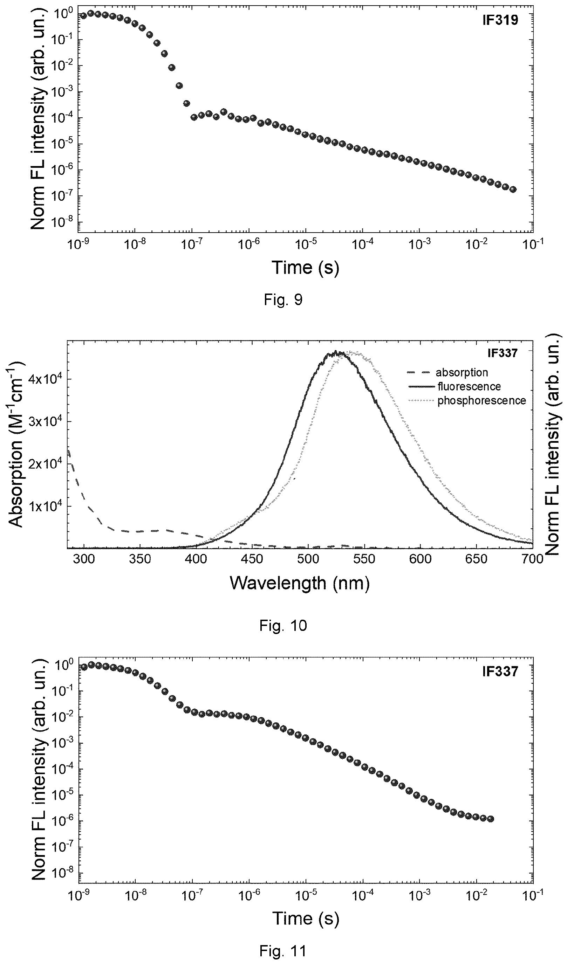

**Photophysical Properties of Some Embodiments.** Absorption and emission spectra of TADF compounds are shown in Figs. 2-13 and the photophysical data is enclosed in Table 2. Lowest-energy absorption peaks, typically of charge-transfer (CT) nature, were observed at 3.30 - 3.56 eV. Molar absorption values were lower for acridine-based TADF compounds, indicating stronger CT character in strongly twisted D-A compounds. Fluorescence spectra of TADF compounds were observed at 2.36 - 2.91 eV, corresponding to blue-green emission color. Efficient spatial separation of HOMO and LUMO determined low singlet-triplet energy gaps ranging in 200 - 510 meV. The lowest singlet-triplet energy gap was found for compound **IF337** (200 meV). All TADF compounds showed an intense delayed fluorescence, which intensity was the largest for compound **IF294** (DF/PF ratio of 11). Fluorescence quantum yield ranged in 0,24 - 0.45 and was the largest for compound **IF294,** having the most intense delayed emission.

**Table 2. Characteristics of emission properties of TADF compounds.**

| **TADF compound** | ***E*_{ABS} (eV)** | ***E*_{PL} (eV)** | **Φ_{PL}** | **DF/PF** | **Δ*E*_{ST} (meV)** |
|---|---|---|---|---|---|
| **IF294** | 3.39 | 2.69 | 0.45 | 11 | 250 |
| **IF306** | 3.30 | 2.59 | 0.39 | 3 | 360 |
| **IF315** | 3.56 | 2.91 | 0.33 | 2 | 320 |
| **IF319** | 3.42 | 2.64 | 0.24 | 3 | 510 |
| **IF345** | 3.57 | 2.82 | 0.34 | 2 | 252 |

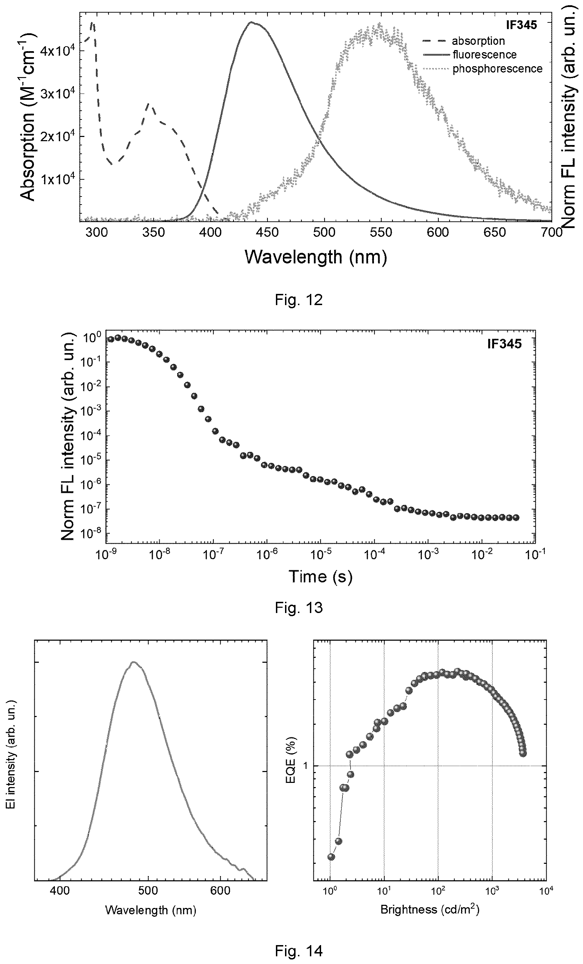

**Electroluminescence properties.** As an illustration of the invention, compound IF294 was used as an emitting layer in OLED device, showcasing the ability to utilize the above-mentioned compounds in electroluminescent devices, presented in Fig. 14. The OLED architecture was selected as following: ITO/TAPC (30 nm)/TcTa (5 nm)/10 wt% tCbz-mPYRs:DPEPO (20 nm)/DPEPO (20 nm)/TmPyPb (50 nm)/LiF (0.8 nm)/AI (100 nm). The device consisted of anode (ITO, indium tin oxide), hole injection layer (TAPC, 1,1-Bis[(di-4-tolylamino)phenyl]cyclohexane), hole transport layer (TcTa, tris(4-carbazoyl-9-ylphenyl)amine), emissive layer (10 wt% doped IF294 in DPEPO, bis[2-(diphenylphosphino)phenyl]ether oxide), electron transport layer (TmPyPb, 1,3,5-Tri(m-pyridin-3-ylphenyl)benzene), electron injection layer (LiF, lithium fluoride) and cathode (Al, aluminium). The device emitted sky blue electroluminescence peaking at about 482 nm. External quantum efficiency (EQE) peaked at about 4.8%.

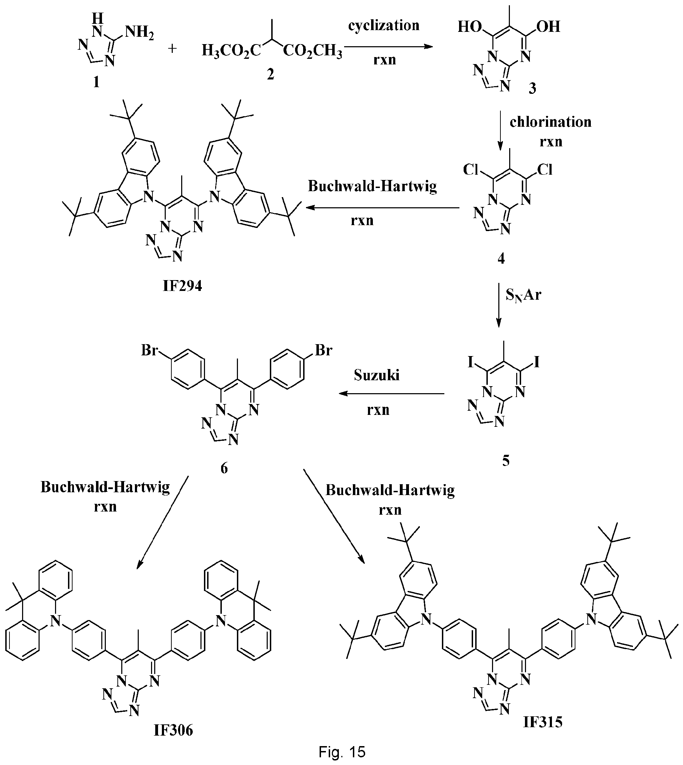

**Synthesis routes of the TADF compounds.** An illustration of synthetic routes for [1,2,4]-triazolo[1,5-a]pyrimidine-based TADF compounds of the invention starting from [1,2,4-triazole-5-amine (1) and substituted alkyl malonate (2) are provided in principal synthesis scheme, in Figure 15. In the reaction of compound 1 with corresponding malonate cyclization reaction occures to give 6-substituted [1,2,4]-triazolo[1,5-a]pyrimidine-5,-7-diol (3). Treatment of compound 3 with phosphorous oxychloride led to substitution of hydroxy groups in compound 3 with chlorine groups to give compound 4 as described in (T. Saito, T. Obitsu, C. Minamoto, T. Sugiura, N. Matsumura, S. Ueno, A. Kishi, S. Katsumata, H. Nakai, M. Toda. Bioorganic and Medicinal Chemistry, 2011, 19, 5955-5966 (http://dx.doi.org/10.1016/j.bmc.2011.08.055)). Further, compound 4 reacted with 3,6-di-tert-butylcarbazole under the Buchwald-Hartwig amination reaction conditions to give 5,7-bis(3,6-di-tert-butylcarbazol-9-yl)-6-methyl-[1,2,4]-triazolo[1,5-a]pyrimidine (IF294). Starting from the same initial 5,7-dichloro-6-methyl-[1,2,4]-triazolo[1,5-a]pyrimidine (4) a general synthetic route for the manufacture of different TADF compounds (IF306, IF315) where donor group is attached to the acceptor [1,2,4]-triazolo[1,5-a]pyrimidine via a phenylene group, consisting of preparation of 5,7-diiodo-6-methyl-[1,2,4]-triazolo[1,5-a]pyrimidine (5), its convertion to 5,7-di(4-bromophenyl)-6-methyl-[1,2,4]-triazolo[1,5-a]pyrimidine (6) employing the Suzuki cross-coupling reaction and following Buchwald-Hartwig amination reaction with 3,6-di-tert-butylcarbazole or 9,9-dimethyl-9,10-dihydroacridine is developed.

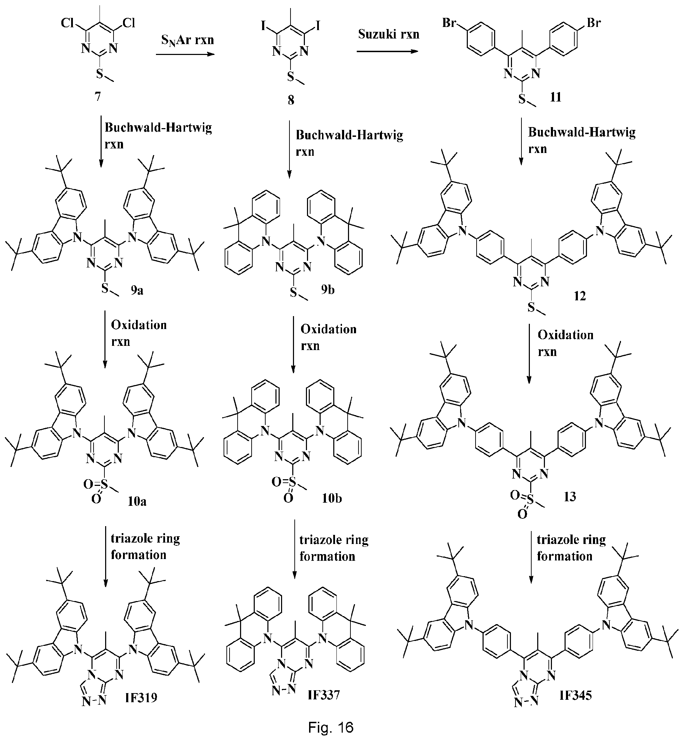

The synthetic routes for the preparation of [1,2,4]-triazolo[4,3-a]pyrimidine-based TADF emitters of the invention is illustrated in Figure 16. 4,6-Dichloro-5-methyl-2-methylthiopyrimidine (7) is utilized as a starting material. Its reaction with hidroiodic acid and potassium iodide at 40 °C led to 4,6-diiodo-5-methyl-2-methylthiopyrimidine (8). Further, 4,6-dichloro-5-methyl-2-methylthiopyrimidine (7) reacted with 3,6-ditert-butylcarbazole or 4,6-diiodo-5-methyl-2-methylthiopyrimidine (8) with 9,9-dimethyl-9,10-dihydroacridine under the palladium-catalyzed Buchwald-Hartwig reaction conditions to give 4,6-bis(3,6-di-tert-butylcarbazol-9-yl)-5-methyl-2-methylthiopyrimide (9a) or 4,6-bis(9,9-dimethyl-9,10-dihydroacridin-10-yl)-5-methyl-2-methylthiopyrimide (9b), correspondingly. Oxidation of compounds 9a, 9b with oxone gave the corresponding 2-methylsulfonylpyrimidines (10a, 10b). Synthesis of 5,7-bis(3,6-di-tert-butylcarbazol-9-yl)-6-methyl[1,2,4]-triazolo[4,3-a]pyrimidine (IF319) and 5,7-bis(9,10-dimethyl-9,10-dihydroacridin-10-yl)-6-methyl[1,2,4]-triazolo[4,3-a]pyrimidine (IF337) was carried out by one pot reaction of the corresponding 2-methylsulfonyl derivatives (10a, 10b) with hydrazine hydrate and following reaction of the intermediate 2-hydrazino derivatives with ethyl orthoformate and their cyclization in the presence of para-methyltoluenesulphonic acid. The synthesis of 5,7-bis(4-(3,6-di-tertbutylcarbazol-9-yl)phenyl)-6-methyl-[1,2,4]-triazolo[4,3-a]pyrimidine (IF345) is carried out from the 4,6-diiodo-5-methyl-2-methylthiopyrimidine (8) including conversion of compound 8 to 4,6-di(4-bromophenyl)-5-methyl-2-methylthiopyrimidine (11) by using palladium-catalysed Suzuki reaction of compound 8 with 4-bromophenylboronic acid; the following cross-coupling reaction with 3,6-di-tert-butylcarbazole to furnish 4,6-bis(4-(3,6-di-tert-butylcarbazol-9-yl)phenyl)-5-methyl-2-methylthiopyrimidine (12); oxidation of methylthio group to give the corresponding 2-methylsulfonyl pyrimidine derivative (13); formation of [1,2,4]triazole ring fused with pyrimidine nucleous by utilizing the one-pot reaction of 2-methylsulfonylpyrimidine 13 with hydrazine hydrate and ethyl orthoformate in the presence of 4-toluenesulfonic acid ( Fig. 16).

More details for manufacture route steps of the synthesized compounds IF294, IF306, IF315, IF319, IF337, and IF345 are disclosed below in description of specific steps of the above synthesis routes (Fig. 15 and Fig 16).

**[1,2,4]-Triazolo[1.5-a]pyrimidine compounds.** Principle synthetic route of [1,2,4]-triazolo[1,5-a]pyrimidine derivatives as TADF compounds of the invention is presented in Figure 15. Explanation that is more detailed is provided below, with yields and spectroscopic characterization information.

### 5,7-Diiodo-6-methyl-[1,2,4]triazolo[1,5-a]pyrimidine (5)

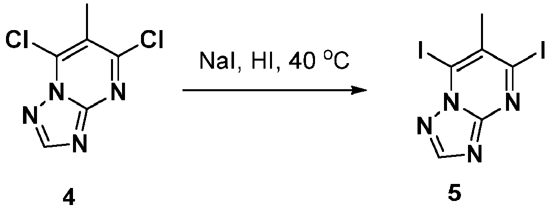

A mixture of 5,7-dichloro-6-methyl-[1,2,4]triazolo[1,5-a]pyrimidine (300 mg, 1,478 mmol) and Nal (443.3 mg, 2.956 mmol, 2 eqv.) in 48 % aqueous HI (4.5 ml) was stirred at 40 °C in the dark for 72h. After completion of the reaction, the mixture was poured into ice/water and neutralized with Na₂CO₃. The aqueous solution was extracted with chloroform (4x25 mL). The combined extract was washed with 5 % aqueous Na₂S₂O₅ (1×25 ml) and water (1×25 ml), then dried with anhydrous Na₂SO₄, filtered and chloroform was removed by distillation under reduced pressure to give the product. 5,7-diiodo-6-methyl-[1,2,4]triazolo[1,5-a]pyrimidine (2). Yield: 426 mg, 74 %. M. p. ≈ 227 °C (dec.). ¹H NMR (CDCl₃, 400 MHz) δ, ppm.: 2.89 (s, 3H, CH₃); 8.48 (s, 1H, CH_{triazol}). ¹³C NMR (CDCl₃, 100 MHz) δ, ppm: 29.95; 104.64; 128.02; 131.22; 154.81 (two C nuclei). HRMS-ESI: m/z calcd. for MH⁺ (C₆H₅I₂N₄): 386.8598; found: 386.8599.

### 5,7-Bis(3,6-di-tert-butyl-9H-carbazol-9-yl)-6-methyl-[1,2,4]triazolo[1,5-a]pyrimidine (IF294).

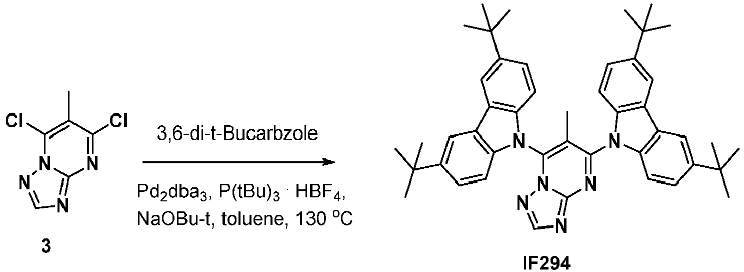

5,7-Dichloro-6-methyl-[1,2,4]triazolo[1,5-a]pyrimidine (1) (80.0 mg, 0.394 mmol, 1 eqv.), 3,6-di-t-butylcarbazole (2.1 eqv.), Pd₂(dba)₃ (25.3 mg, 0.076 mmol, 7 mol%), P(t-Buh·HBF₄ (16 mg, 0.0522 mmol, 14 mol%), NaOtBu (124.8 mg, 1.30 mmol, 3.3 eqv.) and toluene (3 ml) were placed in a screw-cap vial equipped with a magnetic stir bar. The reaction mixture was stirred at 140 °C overnight under argon atmosphere. After completion of the reaction, toluene removed by distillation under reduced pressure, water (40 mL) was added to residue and the aqueous solution was extracted with chloroform (3×25 mL). The combined extract was dried with anhydrous Na₂SO₄, filtered and chloroform was removed by distillation under reduced pressure. Residue was purified by column chromatography using chloroform as an eluent to give the product IF294. Compound recrystallized from 2-propanol. Yield: 90 mg, 33 %. M. p. 224-226 °C. ¹H NMR (CDCl₃, 400 MHz) δ, ppm.: 1.51 (s, 36H, C-CH₃); 1.98 (s, 3H, CH₃); 7.00 (d, J = 8.58 Hz, 2H, CH); 7.51 (d, J = 8.68 Hz, 2H, CH); 7.58 (m, 4H, CH); 8.16 (d, J = 1.59 Hz, 2H, CH); 8.21 (d, J = 1.61 Hz, 2H, CH); 8.53 (s, 1H, CH_{triazol}). ¹³C NMR (CDCl₃, 100 MHz) δ, ppm: 14.51; 31.93; 34.88; 34.92; 110.43; 110.93; 115.22; 116.62; 117.27; 124.23; 124.45; 124.77; 124.91; 136.83; 137.88; 141.16; 145.02; 145.43; 155.03; 155.95; 156.36. HRMS-ESI: m/z calcd. for MH⁺ (C₄₆H₅₃N₆): 689.4326; found: 689.4326.

### 5,7-Bis(4-bromophenyl)-6-methyl-[1,2,4]triazolo[1,5-a]pyrimidine

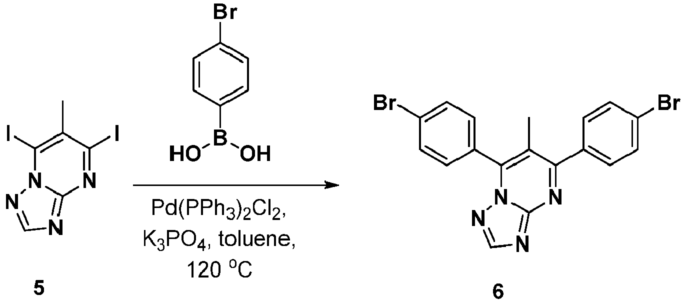

5,7-Diiodo-6-methyl-[1,2,4]triazolo[1,5-a]pyrimidine (5) (108 mg, 0.280 mmol, 1 eqv.), 4-bromophenylboronic acid (135 mg, 0.672 mmol, 2.4 eqv.), Pd(PPh₃)₂Cl₂ (13.7 mg, 0.0196 mmol, 7 mol%), K₃PO₄ (42.71 mg, 2.01 mmol, 7.2 eqv.) and toluene (3 ml) were placed in a screw-cap vial equipped with a magnetic stir bar. The reaction mixture was stirred at 120 °C overnight under argon atmosphere. After completion of the reaction, toluene removed by distillation under reduced pressure, water (40 mL) was added to residue and the aqueous solution was extracted with chloroform (3x25 mL). The combined extract was dried with anhydrous Na₂SO₄, filtered and chloroform was removed by distillation under reduced pressure. Residue was purified by column chromatography using chloroform/EtOAc (5:1) as an eluent to give the product 6. Compound is recrystallized from 2-propanol. Yield: 70 mg, 56 %. M. p. 125-126 °C. ¹H NMR (CDCl₃, 400 MHz) δ, ppm.: 2.29 (s, 3H, CH3); 7.49 (d, J = 8.43 Hz, 2H, CH); 7.58 (d, J = 8.45 Hz, 2H, CH); 7.69 (d, J = 8.44 Hz, 2H, CH); 7.79 (d, J = 8.41 Hz, 2H, CH), 8.43 (s, 1H, CH). ¹³C NMR (CDCl₃, 100 MHz) δ, ppm: 17.16; 116.70; 124.42; 125.54; 128.38; 130.61; 130.65; 131.04; 131.76; 132.47; 153.97; 154.92; 156.08; 164.75. HRMS-ESI: m/z calcd. for MH⁺ (C₁₈H₁₃Br₂N₄): 444.9481; found: 444.9482.

### 5,7-Bis(4-(9,9-dimethyl-9,10-dihydroacridin-10-yl)phenyl)-6-methyl-[1,2,4]triazolo[1,5-a]pyrimidine (IF306) and 5,7-Bis(4-(3,6-di-tert-butyl-9H-carbazol-9-yl)phenyl)-6-methyl-[1,2,4]triazolo[1,5-a]pyrimidine (IF315). General procedure.

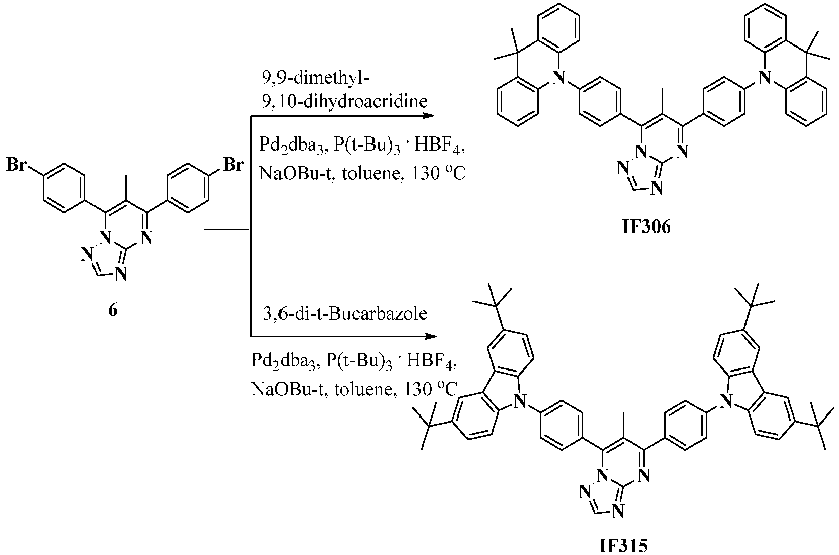

5,7-Bis(4-bromophenyl)-6-methyl-[1,2,4]triazolo[1,5-a]pyrimidine (6) (60.0 mg, 0.135 mmol, 1 eqv.), 10H-9,9-dimethyl-9,10-dihydroacridine (0.284 mmol, 2.1 eqv.) or 3,6-di-t-butylcarbazole ((0.284 mmol, 2.1 eqv.), Pd₂(dba)₃ (8.7 mg, 0.0095 mmol, 7 mol%), P(t-Bu)₃·HBF₄ (5.5 mg, 0.0189 mmol, 14 mol%), NaOtBu (42.8 mg, 0.446 mmol, 3.3 eqv.) and toluene (3 mL) were placed in a screw-cap vial equipped with a magnetic stir bar. The reaction mixture was stirred at 130 °C overnight under argon atmosphere. After completion of the reaction, toluene removed by distillation under reduced pressure, water (40 mL) was added to residue and the aqueous solution was extracted with chloroform (3×25 mL). The combined extract was dried with anhydrous Na₂SO₄, filtered and chloroform was removed by distillation under reduced pressure. Residue was purified by column chromatography using chloroform (IF315) or chloroform/EtOAc (10:1) (IF306) as an eluent to give the product. Compound recrystallized from 2-propanol.

**5,7-Bis(4-(9,9-dimethyl-9,10-dihydroacridin-10-yl)phenyl)-6-methyl-[1,2,4]triazolo[1,5-a]pyrimidine (IF306).** Yield: 57 mg, 33 %. M. p. 183-185 °C. ¹H NMR (CDCl₃, 400 MHz) δ, ppm.: 1.75 (s, 12H, C-CH₃); 2.55 (s, 3H, CH₃); 6.39 (dd, J = 8.03 Hz, J = 1.07 Hz, 2H, CH); 6.46 (dd, J = 8.03 Hz, J = 1.02 Hz, 2H, CH); 6.97-7.10 (m, 8H, CH); 7.50-7.55 (m, 4H, CH); 7.58 (d, J = 8.32 Hz, 2H, CH); 7.68 (d, J = 8.36 Hz, 2H, CH); 7.96 (d, J = 8.34 Hz, 2H, CH); 8.03 (d, J = 8.32 Hz, 2H, CH); 8.59 (s, 1H, CH_{triazol}). ¹³C NMR (CDCl₃, 100 MHz) δ, ppm: 25.37; 31.03; 31.20; 36.05; 36.11; 114.15; 114.37; 116.95; 120.92; 121.19; 125.35; 126.06; 126.44; 126.49; 128.23; 130.31; 130.72; 131.56; 131.68; 131.77; 132.27; 138.28; 140.56; 140.68; 142.87; 143.84; 146.28; 154.17; 156.26; 165.21. HRMS-ESI: m/z calcd. for MH⁺ (C₄₈H₄₁N₆): 701.3387; found: 701.3388.

**5,7-Bis(4-(3,6-di-tert-butyl-9H-carbazol-9-yl)phenyl)-6-methyl-[1,2,4]triazolo[1,5-a]pyrimidine (IF315).** Yield: 66 mg, 58 %. M. p. 251-254 °C. ¹H NMR (CDCl₃, 400 MHz) δ, ppm.: 1.51 (s, 36H, C-CH₃); 2.58 (s, 3H, CH₃); 7.50-7.56 (m, 7H; CH); 7.60 (d, J = 8.64 Hz, 2H, CH); 7.81 (d, J = 8.36 Hz, 2H, CH); 7.92-7.94 (m, 3H, CH); 8.01 (d, J = 8.39 Hz, 2H, CH); 8.18-8.21 (m, 4H, CH); 8.59 (s, 1H, CH_{triazol}). ¹³C NMR (CDCl₃, 100 MHz) δ, ppm: 17.71; 32.00; 34.79; 109.27; 109.36; 116.38; 116.45; 117.23; 123.75; 123.82; 123.85; 123.93; 126.33; 126.49; 127.33; 130.78; 130.98; 131.30; 136.57; 138.61; 138.86; 139.93; 140.83; 143.43; 143.72; 146.38; 153.92; 155.82; 165.38. HRMS-ESI: m/z calcd. for MH⁺ (C₅₈H₆₁N₆): 841.4952; found: 841.4953.

**[1,2,4]-Triazolo[4,3-a]pyrimidines.** The principle synthetic routes [1,2,4]-triazolo[4,3-a]pyrimidine TADF compounds of the invention is provided in Figure 16. Explanation that is more detailed is provided below, with yields and spectroscopic characterization information.

### 4,6-Diiodo-5-methyl-2-methylthiopyrimidine (8).

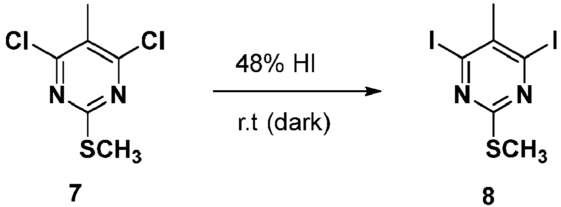

4,6-Dichloro-5-methyl-2-methylthiopyrimidine (4) (240 mg, 1,148 mmol) in 48 % aqueous HI (5 ml) was stirred at 25 °C in the dark for 72h. Water (50 ml) was added to the mixture and the aqueous solution was extracted with chloroform (4×25 mL). The combined extract was washed with 10 % aqueous Na₂CO₃ (2x25 ml), 5 % aqueous Na₂S₂O₅ (1×25 ml) and water (1×25 ml), then dried with anhydrous Na₂SO₄, filtered and chloroform was removed by distillation under reduced pressure. Residue was purified by filtration throughout SiO₂ layer using chloroform:petroleum ether (1:2) as an eluent to give the product 5. Yield: 380 mg (84 %), mp 123-125 °C. ¹H NMR (CDCl3, 400 MHz) δ, ppm.: 2.53 (s, 3H, SCH₃), 2.61 (s, 3H, CH₃). ¹³C NMR (CDCl₃, 100 MHz) δ, ppm: 14.45, 29.57, 132.18, 135.76, 169.97. HRMS-ESI: m/z calcd. for MH⁺ (C₆H₇I₂N₂S): 392.8414; found: 392.8411.

### 4,6-Bis(3,6-di-tert-butyl-carbazol-9-yl)-5-methyl-2-methylthiopyrimidine (6a).

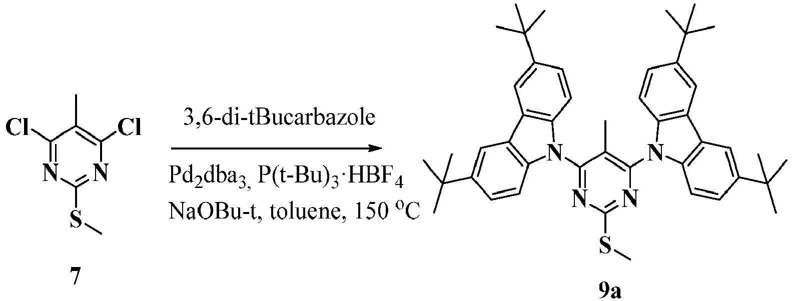

4,6-Dichloro-5-methyl-2-methylthiopyrimidine (7) (120.0 mg, 0.574 mmol, 0.7 eqv.), 3,6-di-tret-butylcarbazole (228.9 mg, 0.820 mmol, 1 eqv.), Pd₂(dba)₃ (26.3 mg, 0.0287 mmol, 5 mol%), P(t-Bu)₃·HBF₄ (16.7 mg, 0.0574 mmol, 10 mol%), NaOtBu (82.7 mg, 0.0861 mmol, 1.5 eqv.) and toluene (3 mL) were placed in a screw-cap vial equipped with a magnetic stir bar. The reaction mixture was stirred at 150 °C for 24 h under argon atmosphere. After completion of the reaction, toluene removed by distillation under reduced pressure, water (40 mL) was added to residue and the aqueous solution was extracted with chloroform (4x25 mL). The combined extract was dried with anhydrous Na₂SO₄, filtered and chloroform was removed by distillation under reduced pressure. Residue was purified by column chromatography using chloroform:petroleum ether (1:2) as an eluent to give the product 6a. Compound recrystallized from 2-propanol. Yield 120 mg (36 %), mp 288-289 °C. ¹H NMR (CDCl3, 400 MHz) δ, ppm.: 1.52 (s, 36H, CH₃(*t*-buthyl)), 1.86 (s, 3H, CH₃), 2.65 (s, 3H, SCH₃), 7.50 (d, J = 8.63 Hz, 4H, CH(Cbz)), 7.61 (dd, J = 8.65 Hz, J = 1.85 Hz, 4H, CH(Cbz)), 8.16 (d, J = 1.52 Hz, 4H, CH(Cbz)). ¹³C NMR (CDCl3, 100 MHz) δ, ppm.: 14.4, 14.9, 32.0, 34.8, 111.0, 115.4, 116.5, 124.0, 124.6, 137.7, 144.5, 160.0, 170.7. HRMS-ESI: m/z calcd. for MH⁺ (C₄₆H₅₄N₄S): 695.4147, found: 695.4144.

### 4,6-Bis(9,9-dimethyl-9,10-dihydroacridin-10-yl)-5-methyl-2-methylthiopyrimidine (9b).

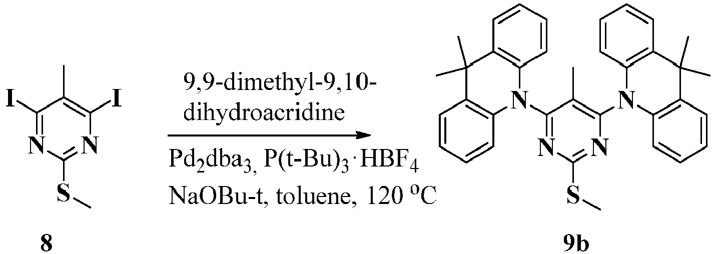

4,6-Diiodo-5-methyl-2-methylthiopyrimidine (8) (162.0 mg, 0.4132 mmol, 1 eqv.), 9,9-dimethyl-9,10-dihydroacridine (190 mg, 0.9091 mmol, 2.2 eqv.), Pd₂(dba)₃ (26.5 mg, 0.02892 mmol, 7 mol%), P(t-Bu)₃·HBF₄ (16.8 mg, 0.05784 mmol, 14 mol%), NaOtBu (130.9 mg, 1.364 mmol, 3.3 eqv.) and toluene (3 ml) were placed in a screw-cap vial equipped with a magnetic stir bar. The reaction mixture was stirred at 120 °C for 24 h under argon atmosphere. After completion of the reaction, toluene removed by distillation under reduced pressure, water (40 mL) was added to residue and the aqueous solution was extracted with chloroform (4x25 mL). The combined extract was dried with anhydrous Na₂SO₄, filtered and chloroform was removed by distillation under reduced pressure. Residue was purified by column chromatography using chloroform:petroleum ether (2:1) as an eluent to give the product 9b. Compound recrystallized from 2-propanol. Yield: 138 mg (60 %), mp 100-101 °C. ¹H NMR (CDCl3, 400 MHz) δ, ppm.: 1.72 (s, 12H, CH₃-acrdn), 1.82 (s, 3H, CH₃), 2.65 (s, 3H, SCH₃), 6.60 (dd, J = 8.12 Hz, J = 1.16 Hz, 4H, CH-acrdn), 7.06 (dt, J = 7.49 Hz, J = 1.24 Hz, 4H, CH-acrdn), 7.13 (dt, J = 7.7 Hz, J = 1.60 Hz, 4H, CH-acrdn), 7.53 (dd, J = 7.76 Hz, J = 1.52 Hz, 4H, CH-acrdn). ¹³C NMR (CDCl3, 100 MHz) δ, ppm: 11.88, 14.54, 31.47, 36.17, 113.84, 122.08, 124.89, 125.90, 126.79, 131.04, 138.13, 164.36, 173.02. HRMS-ESI: m/z calcd. for MH⁺ (C₃₆H₃₅N₄S): 555.2577; found: 555.2571.

### 4,6-Bis(3,6-di-tret-butyl-carbazol-9-yl)-5-methyl-2-methylsulfonylpyrimidine (10a).

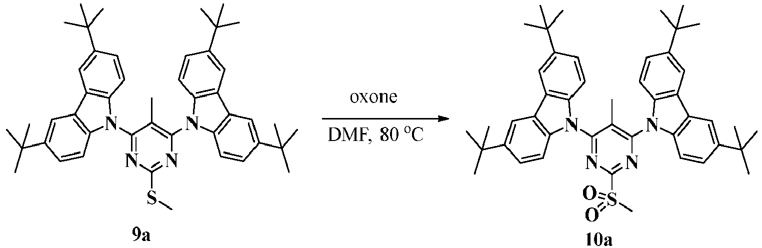

4,6-Bis(3,6-di-*tret*-butyl-carbazol-9-yl)-5-methyl-2-methylthiopyrimidine (9a) (60 mg, 0.0863 mmol, 1 eqv.), oxone (133.2 mg, 2.5 eqv. by active component) and DMF (3 ml) were stirred at 80 °C for 3 h. After completion of the reaction, water (30 mL) was added to reaction mixture and the aqueous solution was extracted with chloroform (3x25 mL). The combined extract was washed with brine twice, dried with anhydrous Na₂SO₄, filtered and chloroform was removed by distillation under reduced pressure. Residue was purified by column chromatography using chloroform/petroleum ether mixture (2:1) as an eluent to give the product 10a. Compound recrystallized from 2-propanol. Yield: 58 mg, 92 %. M. p.: >350 °C. ¹H NMR (CDCl3, 400 MHz) δ, ppm.: 1.52 (s, 36H, (CH₃)₃C); 2.03 (s, 3H, CH₃); 3.45 (s, 3H, CH₃SO₂); 7.54 (d, J = 8.65 Hz, 4H, CH); 7.64 (dd, J = 8.67 Hz, J = 1.89 Hz, 4H, CH); 8.15 (d, J = 1.68 Hz, 4H, CH). ¹³C NMR (CDCl₃, 100 MHz) δ, ppm: 16.54; 31.91; 34.92; 39.41; 111.21; 116.71; 122.61; 124.41; 125.17; 137.29; 145.61; 160.78; 162.53; 163.77. HRMS-ESI: m/z calcd. for MH⁺ (C₄₆H₅₅N₄O₂S): 727.4040; found: 727.4034.

### 4,6-Bis(9,9-dimethyl-9,10-dihydroacridin-10-yl)-5-methyl-2-methylsulfonylpyrimidine (10b).

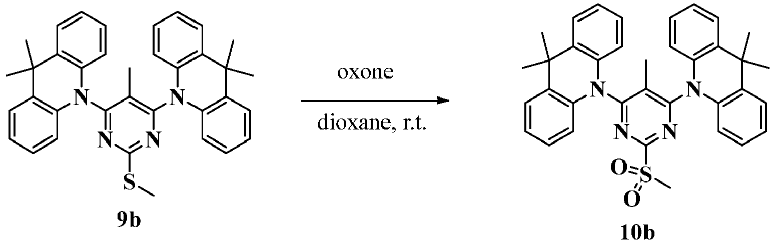

4,6-Bis(9,9-dimethyl-9,10-dihydroacridin-10-yl)-5-methyl-2-methylthiopyrimidine (9b) (40 mg, 0.072 mmol, 1 eqv.), oxone (111.2 mg, 2.5 eqv. by active component) and dioxane (3 ml) were stirred at room temperature for 40 min. After completion of the reaction, water (40 mL) was added to reaction mixture and the aqueous solution was extracted with chloroform (3x25 mL). The combined extract was washed with brine twice, dried with anhydrous Na₂SO₄, filtered and chloroform was removed by distillation under reduced pressure. Residue was purified by column chromatography using chloroform as an eluent to give the product (10b). Compound was recrystallized from 2-propanol. Yield: 25 mg, 59 %. M. p. 238-240 °C. ¹H NMR (CDCl₃, 400 MHz) δ, ppm.: 1.69 (s, 12H, C-CH₃); 1,71 (s, 3H, CH₃); 3.44 (s, 3H, SO₂CH₃); 6.97 (dd, J = 796 Hz, J = 1.28 Hz, 4H, CH); 7.09-7.20 (m, 8H, CH); 7.52 (dd, J = 7.60 Hz, J = 1.64 Hz, 4H, CH). ¹³C NMR (CDCl₃, 100 MHz) δ, ppm: 14.42; 30.15; 36.67; 39.22; 116.21; 123.08; 123.51; 125.51; 126.84; 133.73; 138.53; 164.02; 164.81. HRMS-ESI: m/z calcd. for MH⁺ (C₃₆H₃₅N₄O₂S): 587.2475; found: 587.2466.

### 4,7-Bis(9,9-dimethyl-9,10-dihydroacridin-10-yl)-6-methyl-[1,2,4]triazolo[4,3-a]pyrimidine (IF319) and 4,7-Bis(3,6-di-tert-butyl-9H-carbazol-9-yl)-6-methyl-[1,2,4]triazolo[4,3-a]pyrimidine (IF337). General procedure.

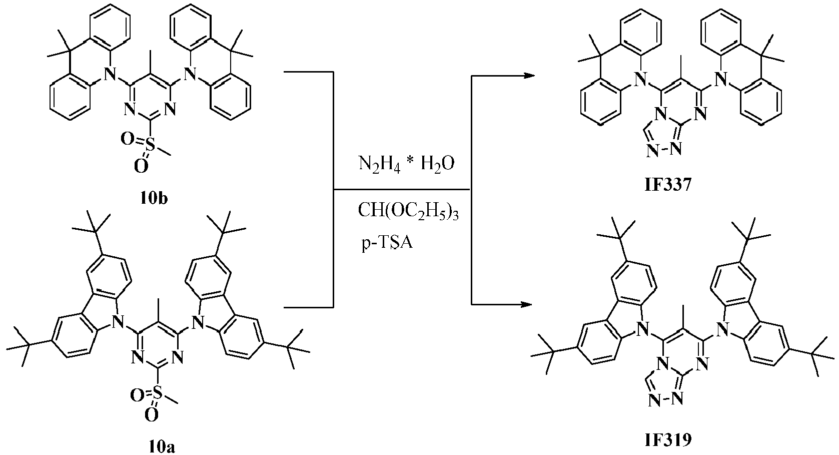

4,6-Bis(hetaryl)-5-methyl-2-(methylsulfonyl)-pyrimidine (10a or 10b) (63 mg), NH₂NH₂·H₂O (50 µl, 96%, 6 eqv. by initial substance) and 2-propanol (5 ml) refluxed for 5 h. After completion of the reaction, the mixture was cooled to room temperature and the resulting precipitate was filtered, washed with 2-propanol and dried. The synthesized 4,6-di-hetaril-5-methyl-pyrimidinyl-2-hydrazine (30 mg, yield: 52-86%) and triethyl orthoformate (0.8 ml) were refluxed for 5-6 h, after that p-toluene sulfonic acid (0.7 mg, 0.075 eqv. by 2-hydrazino-compound) was added and stirred overnight at room temperature. Water (40 mL) was added to reaction mixture and the aqueous solution was extracted with chloroform (3x25 mL). The combined extract was washed with brine twice and dried with anhydrous Na₂SO₄, filtered and chloroform was removed by distillation under reduced pressure. Residue was purified by column chromatography using chloroform as an eluent to give the products IF319 and IF337, respectively. Compounds were recrystallized from 2-propanol.

**4,7-Bis(3,6-ditert-butyl-9H-carbazol-9-yl)-6-methyl-[1,2,4]triazolo[4,3-a]pyrimidine** (IF319). Yield: 15 mg, 21 %. M. p. >300 °C. ¹H NMR (CDCl₃, 400 MHz) δ, ppm.: 1.49 (m, 36H, C-CH₃); 1.94 (s, 3H, CH₃); 7.04 (d, J = 8.57 Hz, 2H, CH); 7.55-7.64 (m, 6H, CH); 8.15 (s, 2H, CH); 8.24 (d, J = 1.61 Hz, 2H, CH); 8.35 (s, 1H, CH_{triazol}). ¹³C NMR (CDCl₃, 100 MHz) δ, ppm: 14.12; 31.89; 31.91; 34.89; 34.99; 109.83; 111.20; 115.22; 116.64; 117.55; 124.28; 124.89; 125.05 (two nucleuses); 133.09; 136.24; 136.90; 137.82; 145.28; 146.11; 153.27; 155.79. HRMS-ESI: m/z calcd. for MH⁺ (C₄₆H₅₃N₆): 689.4326; found: 689.4326.

**4,7-Bis(9,9-dimethyl-9,10-dihydroacridin-10-yl)-6-methyl-[1,2,4]triazolo[4,3-a]pyrimidine (IF337).** Yield: 25 mg, 34 %. M. p. 156-158 °C. ¹H NMR (CDCl₃, 400 MHz) δ, ppm.: 1.68 (s, 3H, CH₃); 1.81 (s, 6H, C-CH₃); 1.83 (s, 6H, C-CH₃); 6.16 (d, J = 7.79 Hz, 2H, CH); 7.06-7.25 (m, 8H, CH); 7.37 (d, J = 7.89 Hz, 2H, CH); 7.58 (dd, J = 12.65 Hz, J = 7.60 Hz, 4H, CH); 8.42 (s, 1H, CH_{triazol}). ¹³C NMR (CDCl₃, 100 MHz) δ, ppm: 12.73; 32.15; 33.20; 35.93; 36.83; 112.03; 116.61; 121.31; 123.62; 123.97; 125.58; 127.00; 127.23; 127.81; 130.72; 132.61; 133.93; 134.84; 138.76; 139.23; 158.93. HRMS-ESI: m/z calcd. for MH⁺ (C₃₆H₃₃N₆): 549.2761; found: 549.2761.

### 4,6-Bis(4-bromophenyl)-5-methyl-2-methylthiopyrimidine (11).

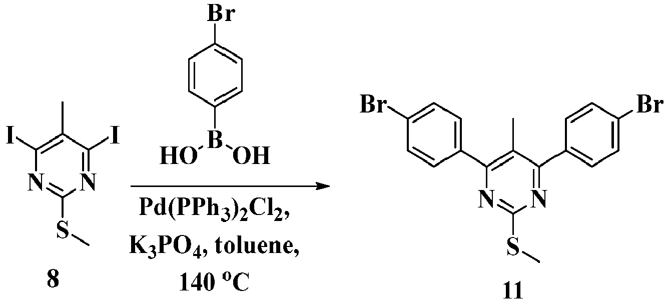

4,6-Diiodo-5-methyl-2-methylthiopyrimidine (8) (120 mg, 0.306 mmol, 1 eqv.), 4-bromophenylboronic acid (147.6 mg, 0.735 mmol, 2.4 eqv.), Pd(PPh₃)₂Cl₂ (17.6 mg, 0.0153 mmol, 5 mol%), K₃PO₄ (467.4 mg, 2.204 mmol, 7.2 eqv.) and toluene (5 ml) were placed in a screw-cap vial equipped with a magnetic stir bar. The reaction mixture was stirred at 140 °C overnight under argon atmosphere. After completion of the reaction, toluene was removed by distillation under reduced pressure, water (40 mL) was added to residue and the aqueous solution was extracted with chloroform (3x25 mL). The combined extract was dried with anhydrous Na₂SO₄, filtered and chloroform was removed by distillation under reduced pressure. Residue was purified by column chromatography using chloroform/petroleum ether (1:1) as an eluent to give the product 11. Compound recrystallized from 2-propanol. Yield: 94 mg, 68 %. M. p. 148-150 °C. ¹H NMR (CDCl₃, 400 MHz) δ, ppm.: 2.30 (s, 3H, CH₃); 2.63 (s, 3H, SCH₃); 7.56 (d, J = 8.37 Hz, 4H, CH); 7.66 (d, J = 8.33 Hz, 4H, CH). ¹³C NMR (CDCl₃, 100 MHz) δ, ppm: 14.26; 17.24; 120.10; 123.90; 130.83; 131.56; 137.27; 166.13; 169.21. HRMS-ESI: m/z calcd. for MH⁺ (C₁₈H₁₄Br₂N₂S): 447.9244; found: 447.9297.

### 4,6-Bis(4-(3,6-di-tert-butyl-9H-carbazol-9-yl)phenyl)-5-methyl-2-methylthiopyrimidine (12).

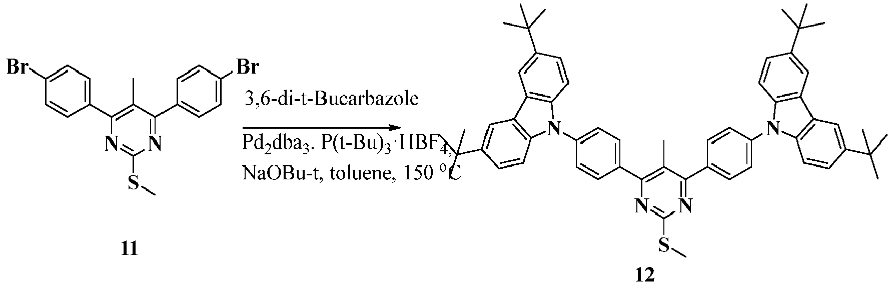

4,6-Bis(4-bromophenyl)-5-methyl-2-(methylthio)pyrimidine (11) (76.5 mg, 0.170 mmol, 1 eqv.), 3,6-di-tret-butylcarbazole (104.3 mg, 0.374 mmol, 2.2 eqv.), Pd₂(dba)₃ (7.8 mg, 0.0085 mmol, 5 mol%), P(t-Bu)₃·HBF₄ (4.9 mg, 0.017 mmol, 10 mol%), NaOtBu (53.9 mg, 0.561 mmol, 3.3 eqv.) and toluene (3 mL) were placed in a screw-cap vial equipped with a magnetic stir bar. The reaction mixture was stirred at 150 °C for 24 h under argon atmosphere. After completion of the reaction, toluene removed by distillation under reduced pressure, water (30 mL) was added to residue and the aqueous solution was extracted with chloroform (3x25 mL). The combined extract was dried with anhydrous Na₂SO₄, filtered and chloroform was removed by distillation under reduced pressure. Residue was purified by column chromatography using chloroform:petroleum ether (from 1:2 to 1:1) as an eluent to give the product 12. Compound recrystallized from 2-propanol. Yield: 90 mg, 63 %. M. p. 323-326 °C. ¹H NMR (CDCl₃, 400 MHz) δ, ppm.: 1.51 (s, 36H, C-CH₃); 2.60 (s, 3H, SMe); 2.78 (s, 3H, CH₃); 7.52-7.55 (m, 8H, CH); 7.78-7.84 (m, 4H, CH_{Ph}); 7.98-8.04 (m; 4H, CH_{Ph}); 8.18-8.20 (m, 4H, CH). ¹³C NMR (CDCl₃, 100 MHz) δ, ppm: 14.42; 17.80; 32.01; 34.79; 109.33; 116.37; 123.73; 123.80; 125.31; 126.20; 131.10; 135.46; 138.85; 139.96; 143.40; 166.30; 168.63. HRMS-ESI: m/z calcd. for MH⁺ (C₅₈H₆₃N₄S): 847.4768; found: 847.4755

### 4,6-Bis(4-(3,6-di-tert-butyl-9H-carbazol-9-yl)phenyl)-5-methyl-2-methylsulfonylpyrimidine (13).

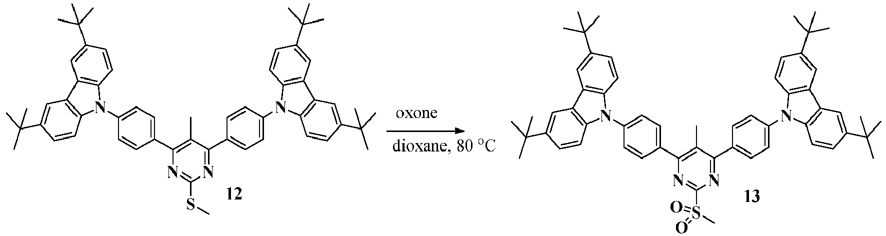

4,6-Bis(4-(3,6-di-tert-butyl-9H-carbazol-9-yl)phenyl)-5-methyl-2-methylthiopyrimidine (12) (165 mg, 0.1948 mmol, 1 eqv.), oxone (300.6 mg, 2.5 eqv. by active component) and dioxane (3 ml) were stirred at 80 °C for 3 h. After completion of the reaction, water (40 mL) was added to the reaction mixture and the aqueous solution was extracted with chloroform (3x25 mL). The combined extract was washed with brine twice, dried with anhydrous Na₂SO₄, filtered and chloroform was removed by distillation under reduced pressure. Residue was purified by column chromatography using chloroform as an eluent to give the product 13. Compound was recrystallized from 2-propanol. Yield: 65 mg, 38 %. M. p. 301-303 °C. ¹H NMR* (CDCl₃, 400 MHz) δ, ppm.: 1.51 (s, 36H, C-CH₃); 2.74 (s, 3H, CH₃); 3.14 (s, 3H, SO₂CH₃); 7.49-7.56 (m, 8H, CH); 7.80 (d, J = 8.70 Hz, 4H, CH_{Ph}); 8.02 (d, J = 8.70 Hz, 4H, CH_{Ph}); 8.19 (s, 4H, CH). ¹³C NMR* (CDCl₃, 100 MHz) δ, ppm: 18.48; 32.00; 34.78; 40.32; 109.25; 116.40; 123.77; 123.82; 126.32; 126.59; 131.15; 135.59; 138.83; 140.12; 143.47; 167.84; 170.66. HRMS-ESI: m/z calcd. for MH⁺ (C₅₈H₆₃N₄O₂S): 879.4666; found: 879.4649.

### 5,7-Bis(4-(3,6-di-tert-butyl-9H-carbazol-9-yl)phenyl)-6-methyl-[1,2,4]triazolo[4,3-a]pyrimidine (IF345).

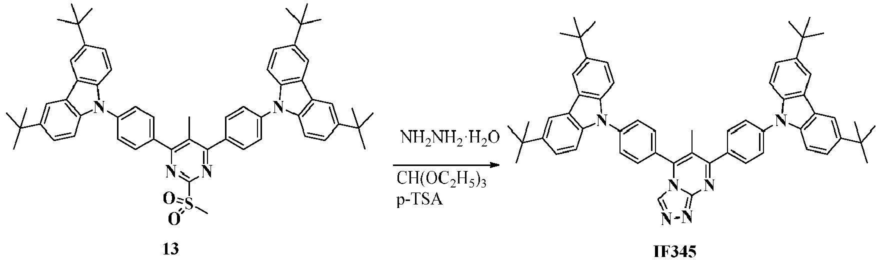

4,6-Bis(4-(3,6-di-tert-butyl-9H-carbazol-9-yl)phenyl)-5-methyl-2-methylsulfonylpyrimidine (13) (46.8 mg, 0.0532 mmol), NH₂NH₂·H₂O (26 µl, 96%, 10 eqv. by initial substance) and 2-propanol (5 ml) refluxed for 5 h. After completion of the reaction, the mixture was cooled to room temperature and the resulting precipitate was filtered, washed with 2-propanol and dried. The synthesized 9,9'-((2-hydrazinyl-5-methylpyrimidine-4,6-diyl)bis(4,1-phenylene))bis(3,6-di-tert-butyl-9H-carbazole) (40 mg, yield: 90 %) and triethyl orthoformate (1 ml) were refluxed for 6 h, after that p-toluenesulfonic acid (0.8 mg, 0.075 eqv. by hydrazinyl-compound) was added and stirred overnight at room temperature. Water (40 mL) was added to reaction mixture and the aqueous solution was extracted with chloroform (3x25 mL). The combined extract was washed with brine twice and dried with anhydrous Na₂SO₄, filtered and chloroform was removed by distillation under reduced pressure. Residue was purified by column chromatography using chloroform as an eluent to give the product IF345. Compound recrystallized from 2-propanol. Yield: 42 mg, 77 %. M. p. 260-263 °C. ¹H NMR (CDCl₃, 400 MHz) δ, ppm.: 1.50-1.53 (m, 36H, C-CH₃); 2.48 (s, 3H, CH₃); 7.49-7.58 (m, 8H, CH); 7.78-7.88 (m, 4H, CH), 7.96-8.02 (m, 4H, CH); 8.20 (d, J = 8.91 Hz, CH); 8.62 (s, 1H, CH_{triazol}). ¹³C NMR (CDCl₃, 100 MHz) δ, ppm: 17.09; 31.97; 31.99; 34.77; 34.82; 109.15; 109.27; 116.38; 116.58; 116.85; 123.78; 123.82; 124.00; 124.08; 126.29; 127.37; 127.70; 130.38; 130.67; 133.13; 136.22; 138.49; 138.84; 140.11; 141.38; 141.46; 143.46; 144.03; 152.91; 165.53. HRMS-ESI: m/z calcd. for MH⁺ (C₅₈H₆₁N₆): 841.4952; found: 841.4952.

## Claims

1. An organic thermally activated delayed fluorescence (TADF) compound, comprising a [1,2,4]-triazolo[1,5-a]pyrimidine or [1,2,4]-triazolo[4,3-a]pyrimidine TP group,
**wherein** the TP group is according to any one of formulas **Ia** and **Ib** 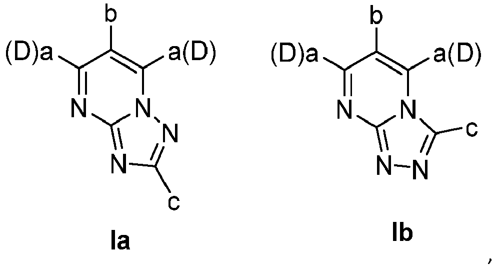
• wherein the **-b** group is any one of:
∘ hydrogen -H,
∘ substituted or unsubstituted primary, secondary or tertiary alkyl, preferably, of C1-C10 order;
∘ cycloalkyl,
∘ alkenyl;
• wherein the -c group is any one of:
∘ hydrogen -H,
∘ substituted or unsubstituted primary, secondary or tertiary, non-cyclic or cyclic alkyl, preferably, of C1-C10-order,
∘ substituted or unsubstituted aryl;
• wherein each of **-a(D)** groups is donor group **(D)** or aryl optionally substituted with donor groups **(D)** wherein said donor group **(D)** is heteroaryl substituted with any of
∘ hydrogen -H,
∘ substituted or unsubstituted primary, secondary or tertiary alkyl, preferably, of C1-C10 order, and more preferably, of C1-C4 order,
∘ substituted or unsubstituted aryl or heteroaryl,
∘ amino group NH₂, NHR or NR² wherein the substituents R at the nitrogen in the amino group are any one of
∘ substituted or unsubstituted aryl or heteroaryl.

2. The TADF compound according to claim 1, **wherein** 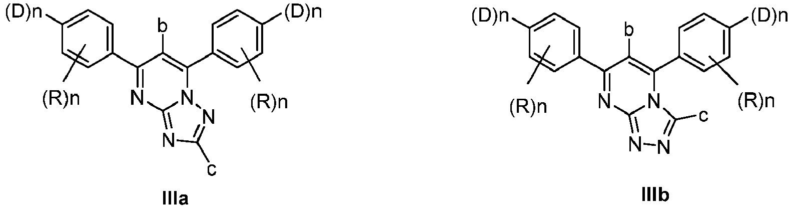 each group **-a(D)** in formulas **Ia** and **Ib** comprises a phenylene spacer, binding the TP group to:
• n of heteroaryl donor **(D)** groups **(D)n-,** wherein each donor **(D)** group is bonded to carbon atom of the benzene ring, wherein n is at least 1 for one of the benzene rings;
• **(R)n** groups, where n is from 0 to 4, each one of **(R)n** groups is bonded to carbon atom of the benzene ring and is any one of:
∘ hydrogen -H,
∘ substituted or unsubstituted primary, secondary or tertiary alkyl, preferably, of C1-C4 order.

3. The TADF compound according to claims 1 to 2, **wherein** said donor groups (D) are heteroaryl groups any of 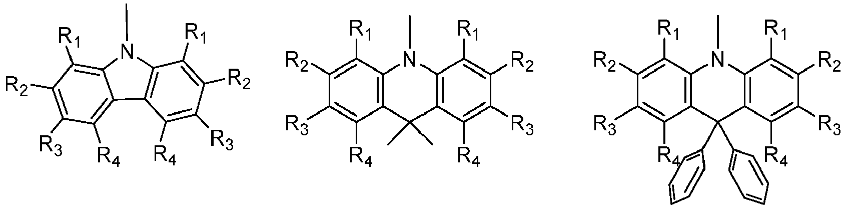 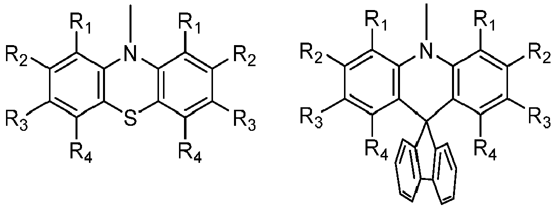 wherein each group of R₁, R₂, R₃, R₄, independently of its occurrence in the donor (D) group, is any of
• hydrogen -H,
• substituted or unsubstituted primary, secondary or tertiary alkyl, preferably, of C1-C10 order, and more preferably, of C1-C4 order,
• substituted or unsubstituted aryl or heteroaryl,
• amino group NH₂, NHR or NR², wherein the substituents R at the nitrogen in the amino group are any one of
∘ substituted or unsubstituted aryl or heteroaryl.

4. The TADF compound according to any one of claims 1 to 3, **wherein** according to claim 4, donor groups (D) are of the form 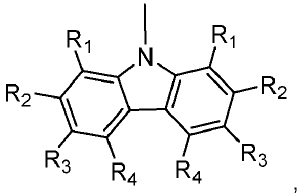 wherein at least one of said donor (D) groups is the donor (D) of the form

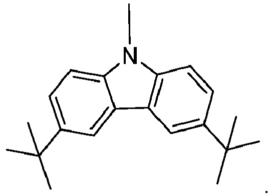

5. The TADF compound according to any one of claims 1 to 3,
w her e i n according to claim 4, donor groups (D) are of the form 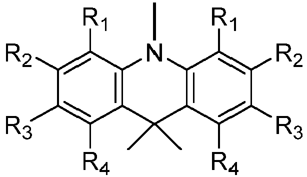 wherein at least one of said donor (D) groups is the donor (D) of the form

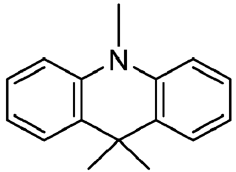

6. The TADF compound according to any claims 1 to 5, **wherein** the preferred TADF compounds are from the group of IF294, IF306, IF315, IF319, IF337, IF345 compounds

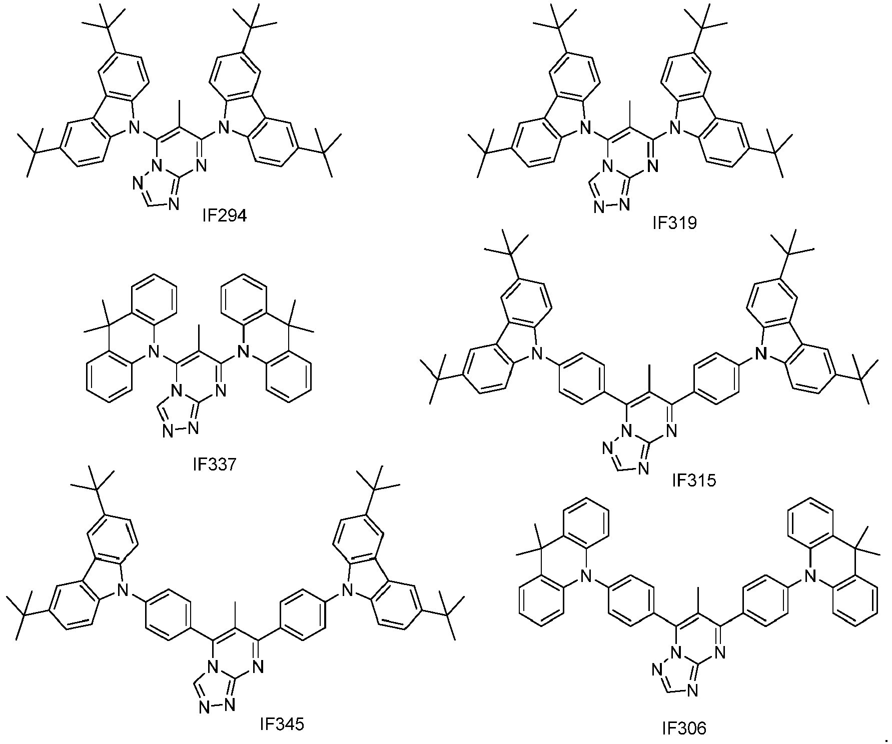

7. A process of preparation of TADF compounds according to Claims 1 to 6, **characterized in that** the process of preparation comprises at least
• a step of modification of [1,2,4]-triazolo[1,5-a]pyrimidine and [1,2,4]-triazolo[4,3-a]pyrimidine heterocycles at the position 6 with alkyl groups in the initial steps of the process,
• and a step of formation of [1,2,4]-triazolo[4,3-a]pyrimidine moiety from corresponding 2-methylsulfonylpyrimidines by three-step one-pot process of preparation, in the last step of reaction sequences.

8. Use of TADF compounds according to Claims 1 to 6, **wherein** said TADF compounds are for OLED and light-emitting applications to generate fluorescence spectra corresponding to blue-cyan-green emission color at the range 2.36 - 2.91 eV.
